# NEW EUROPEAN PATENT SPECIFICATION

(11) **EP 2 138 055 B2**
(45) Date of publication and mention of the opposition decision: **02.03.2016**
(45) Mention of the grant of the patent: 29.08.2012
(21) Application number: 09161242.4
(22) Date of filing: 27.05.2009
(51) Int. Cl.: A61K 9/48, A61K 45/06, A21D 8/04, A61K 31/04, A61K 31/07, A61K 33/30, A23D 7/005, A23D 9/007, A21D 2/14, A23C 9/152, A21D 2/02, A23L 5/42, A23L 33/00, A23L 21/00, A23L 33/16, A21D 2/00, A23L 2/00, A23L 33/10

(54) **Formula food to be beneficial for visuognosis persistence and use thereof**
Für die Visuognose förderliche Lebensmittelzusammensetzung und dessen Verwendung
Formule d'aliment bénéfique pour la persistance de visuognosis et utilisation associée

(30) Priority: 02.06.2008 CN 200810110542
(43) Date of publication of application: 30.12.2009
(73) Proprietor: Zhejiang Medicine Co., Ltd. Xinchang Pharmaceutical Factory, Zhejiang 312500 (CN)
(72) Inventor: Xu, Xinde, Xinchang County Zhejiang 312500 (CN); Zhou, Di, Xinchang County Zhejiang 312500 (CN); Zhang, Lihua, Xinchang County Zhejiang 312500 (CN); Shao, Bin, Xinchang County Zhejiang 312500 (CN)
(74) Representative: Gervasi, Gemma

(56) References cited:
- EP-A- 1 516 542
- EP-A- 1 920 711
- EP-A1- 0 143 705
- WO-A-01/19383
- WO-A-02/47493
- WO-A-2007/076416
- WO-A2-2005/006890
- WO-A2-2007/050521
- DE-U1- 20 300 305
- DE-U1-202006 014 588
- US-A1- 2005 136 130
- US-A1- 2005 249 821
- US-A1- 2007 082 064
- US-A1- 2007 166 354
- US-B1- 7 282 225
- O'CONNELL: 'Macular Carotenoids and Age-related Maculopathy' ANN MED. SINGAPORE 2006 November 2006,
- ANNETT HORMANN: 'Diplomarbeit Fachbereich Ökotrophologie', February 2006 deel 'Lutein und Zeaxanthin'

## Description

### FIELD OF THE INVENTION

The present invention relates to a formula food added with several kinds of components to be beneficial for human eye health ,in order to provide supplements for indispensable materials such as lutein, zeaxanthin, zinc and selenium necessary for human eyes. The present invention belongs to the field of food processing.

### BACKGROUND OF THE INVENTION

Eye health is a permanent and important topic. The focus on eye health has never stopped. Currently, various of eye protection products have present on the market. People have been trying to prevent or treat all kinds of eye discomfort or ophthalmic disease, such as asthenopia, amblyopia, myopia, cataract, age-related macular degeneration (ARMD) and the like, and keep eyes healthy by absorbing or using some natural or synthetic substances or some trace elements. Although the effects of using these products are different, people have not stopped to seek for the optimal medical compositions to achieve the best effect on eye protection.

Among all of eye protection components, lutein and zeaxanthin are two very important substances. Lutein and zeaxanthin are natural carotenoids which widely exist in vegetables, flowers, fruits and some algae living creatures. They are widely used in many fields such as food, health products, cosmetic products, medicine, tobacco, feed for animals and poultries. Recently researches have found that lutein and zeaxanthin not only have a close relationship with a disease of age-related macular degeneration (ARMD), but also can effectively prevent from visual failure. Lutein and zeaxanthin are two only carotenoids existing in the human retina, which are selectively deposited in the macular region and the whole retina, generally there is the highest density around the macular fovea and a gradually decreased density around the retina. These macular pigments are able to effectively prevent from occurrence of the oxidation reaction in the retina. It can be assumed that an effective method of treating and preventing the macular degeneration or injury is to supplement lutein and zeaxanthin. Furthermore, lutein and zeaxanthin can be used to improve the visual efficacy in the absence of ARMD, for example, there are some good healthcare functions for the juvenile myopia, senile blindness caused by muscle degradation, and the eye damage caused by the ultraviolet radiation from sunlight and computer. A lot of clinical testing results show that the amount of recommended ingestion of lutein and zeaxanthin for human body is 6 mg per day. It is generally thought that the acceptable daily intake (ADI) of lutein crystal is 25mg/person/day, which can not result in the carotenoid yellow dermatosis.

More and more people are interested in the special physiological functions of lutein and zeaxanthin. Currently, a large amount of health food and fortified food containing the above-mentioned two natural pigments have present abroad. The FDA of USA approved the use of lutein and zeaxanthin as food supplement agents in foods in 1995 to improve nutritive values, for example, the use in beverage to be beneficial for eyesight and in infant food. At the end of 2006, the use of lutein as coloring agent in beverage, baked food, frozen food, and food such as fruit jelly and fruit jam with the addition amount of 50∼150mg/kg, has been also approved in China. In the meanwhile, the use of lutein as nutrition enhancement agent has been approved as well. The impetus of new market makes the market value of lutein reach 139 million dollars in 2004, but only 64 million in 1999. The tendency of this strong increasing is still continued, and BBC thinks that the increasing rate will reach 6.1 % every year by 2009.

In addition to lutein and zeaxanthin, other substances such as zinc and selenium are beneficial to eye health. Studies show that zinc can increase sensitivities of optic nerves, and is the main component of the retinal reductase in the retinal cells. The enhancement of zinc can directly affect synthesis and metabolism of xanthopsin from vitamin A, which may be helpful for the effect of retinal, thereby to increase human adaptability to weak light. The lack of zinc may also affect the ability of retinal cell to distinguish from colors. Selenium is an important trace element to maintain eyesight, and is the main component of glutathione peroxidase. The lack of selenium may lead to amblyopia. Accordingly, the carophylls such as lutein, zeaxanthin and zinc, selenium are good partners. The combination of the four components has the effects on promotion enhancement, and has effects on protection of eyes, prevention of juvenile myopia, sthenopia among workers and senile visual failure.

In view of further attention to eye health and the aforementioned identification for eye protection function, foods including lutein or selenium or zinc or three of them, such as beverage, milk, yoghurt, and the like, have also present in the market, but the function or addition of zeaxanthin have been generally ignored in these products. Actually, in some sense, zeaxanthin has the same, or even better, eye protection effects as lutein. For example, at the macular of the human retinal central, the content of zeaxanthin is generally about two times of that of lutein. The nearer the edge of the retinal is, the higher the content of lutein is, and the relatively lower the content of zeaxanthin is. At the most edge of the retinal, the content of lutein is about three times of that of zeaxanthin, in the human plasma, the content of lutein is also about three times of that of zeaxanthin, it shows that there is a transfer mechanism from lutein to zeaxanthin in human body. Meanwhile, other studies also found that some of lutein ingested in by human body will change into zeaxanthin. Accordingly, zeaxanthin plays non-negligible roles in eye health of human. The results of our preliminary test in human body also proved that visual performances of human eyes, especially visuognosis persistence and eye consciousness symptoms may be well improved, when being supplemented with the mixture of lutein and zeaxanthin added with selenium and zinc.

As described above, there are various eye protection products in the market, however, most of them are compositions containing one, or two, or three components of the four. For example, CN1625395A relates to the composition of lutein and zeaxanthin used for glare protection; CN1253091 relates to a dairy product or beverage to be beneficial for eyesight containing lutein, zinc and selenium; US2005/0147648 keeps eyes healthy by supplementing a combination of zeaxanthin, polyunsaturated fatty acids, plant extracts and the like; US7,267,830 relates to the objective of improving eye health level by supplementing carotenoids and vitamin A, C, DHA and some trace elements, wherein in the disclosed formulation, the proportion of lutein and zeaxanthin is kept at the level of 3: 1, and the function of zeaxanthin is not specially mentioned; US7,282,225 supplements vitamin A, C, E, carotenoids (beta-carotenoid, lutein, zeaxanthin), trace elements by dietary to improve visual performance and acuity, but the unique effect of zeaxanthin on improving visuognosis persistence is not highlighted in this formulation.

WO 01/19383 discloses a dietary supplement for ocular diseases or disorders such as glaucoma, age related macular degeneration, retinal ischemia comprising 85 mg beadlets, 20 mcg selenium, 30 mg zinc, 200 mg vitamin C and 75 IU vitamin E wherein the beadlets comprise 3% w/w lutein/zeaxanthin (the ratio of lutein: zeaxanthin is 1:0.05-0,05:1).

DE 20 2006 014588 discloses a carotenoid-containing product suitable for complementing the nutrition in age-related eye diseases comprising one capsule and one solution, the solution comprising 15 mg zinc, 60 µg selenium, 200 mg vitamin C and 73 mg vitamin E and the capsule comprising 12 mg Lutein and 840 µg zeaxanthin (the ratio of lutein: zeaxanthin is 14,3:1).

US 2007/166354 discloses a milk based ready to feed, infant formula improved retinal health and vision development and reduces the risk or severity of retinopathy of prematurity in the infants which comprises in 454 kg an amount 90 µg/L lutein with zeaxanthin and 5 g of a vitamin premix which contains 6,7 g zinc sulfate per 65 g and 0,025 g sodium selenate an 21 g of a vitamin ADEK premix (comprising 4 g alpha-tocopherol). The lutein/zeaxanthin mixture contains 1,25% zeaxanthin by weight of lutein.

US-B1-7 282 225 discloses a dietary supplement composition for improving retinal health comprising 15-30% by weight vitamin E, 10-25% zinc, 0,05-0,5% selenium, 2-20% lutein and 0,1-1% zeaxanthin (the ratio of lutein: zeaxanthin is 2:1-200:1).

DE 203 00 305 discloses a micronutrient combination product for the dietetic prevention and treatment of eye diseases especially age-related macular degeneration which contains 0,3-2,4 g lutein, 0,025 -0,2 g zeaxanthin, 2,5-20 g vitamin C, 0,4-4 g vitamin E, 1-4 mg selenium and 0,25-2 g zinc per 100 g (the ratio of lutein to zeaxanthin ranges between 1,5:1 and 96:1).

The present invention provides a formula food containing the aforementioned four components in a certain proportion, as specified in the subject-matter of claim 1. Such food provides indispensable trace elements for human eye health, such as lutein, zeaxanthin, zinc, and selenium, and is conducive to the human eye health, especially is beneficial for the improvement of visuognosis persistence of human eyes, helpful for the asthenopia remission and improvement of eye consciousness symptoms.

### SUMMARY OF THE INVENTION

Lutein and zeaxanthin are the only two kinds of carotenoids existing in the human retina, which are closely related to the macular degeneration. Zinc and selenium can increase adaptabilities to weak light and color distinguishing ability of human eyes. The compositions containing the aforementioned four substances in a certain proportion are beneficial for human eye health when being used in formula food. The objective of the present invention is to describe the formula food including the composition described above.

As used herein, the term "visual performance" is referred to as visual function such as acuity, contrast sensitivity, glare recovery, dark adaptation, visuognosis persistence, light stress recovery and color vision and the like, wherein special attention is the increase of visuognosis persistence, such as asthenopia remission and improvement of eye consciousness symptoms.

As used herein, the term "visuognosis persistence" is an index for evaluating asthenopia. When the cortical excitability in the human brain reduces, the visual analysis function decreases, in the process of eyes fixating at object, invisible duration will increase and visuognosis duration will decrease. The percentage of visuognosis duration to fixation duration is referred to as visuognosis persistence, which is an index to comprehensively reflect the visual performance and psychological function. The visuognosis persistence (%) = visuognosis duration/ total fixation duration* 100.

As defined herein, "formula food" is referred to as various foods for enhancing certain nutritional function, such as formula milk powder, including infant formula milk powder or senile formula milk powder and the like; various functional beverages such as beverages containing milk, orange juice beverages, coke, carbonated beverages, suspended beverages, and so on; baked food such as cakes, biscuits and so on, and, etc. including various dietary supplement tablet, capsule for nutrition supplements.

The present invention provides a formula food beneficial for eye health, comprising lutien, zeaxanthin, zinc, and selenium.

As defined herein, "eye health" is referred to as good effect of increasing of visuognosis persistence and asthenopia remission and improvement of eye consciousness symptoms as well as preventing from or treating Agerelated Macular Degeneration (ARMD).

Wherein, the zinc is inorganic zinc salt or organic zinc salt. The inorganic zinc salt is zinc sulfate or zinc sulfite; the organic zinc salt is zinc orotate or zinc ascorbate.

The selenium is inorganic selenium salt or organic selenium salt. The inorganic selenium salt is selenophosphate, or selenite. The organic selenium salt is selenoamino acid or selenium yeast or selenium-enriched yeast. Wherein, the selenophosphate is sodium selenophosphate; the selenite is sodium selenite or sodium selenate; the Selenoamino acid is L-selenomethionine; the selenium yeast or selenium-enriched yeast is Brewer's yeast or Barker's yeast.

Wherein the amount of the lutein added in the formula food is 0.3-250mg/kg. The amount of the zeaxanthin added in the formula food is 0.3-250mg/kg. Preferably, the amount of the lutein added is 2.0-80mg/kg in the formula food; the amount of the zeaxanthin added is 2.0-80mg/kg in the formula food. According to the present invention, the proportion of mass of lutein and zeaxanthin is in the range of 0.1:2.0-1.0: 1.0. In particular, the lutein and zeaxanthin of the present invention may be obtained by mixing in a certain proportion, that is, they have been mixed in a certain proportion before the dosage available in preparation to add into the formula food is obtained.

Wherein the amount of zinc added in the formula food is 4-80mg/kg which is based on the weight of pure zinc when the added zinc is organic or inorganic zinc. The amount of selenium added in the formula food is 30-350µg/kg which is based on the weight of pure selenium when the added selenium is organic or inorganic selenium.

Wherein the lutein exists in the form of crystalloid or aliphatic ester is extracted from natural sources. The dosage form of the lutein added is microencapsulated powder, or homogeneously dispersed in edible vegetable oils, or aqueous dispersible emulsion Wherein the mass percent of the lutein in the corresponding dosage form is 1-25 wt%.

Wherein the zeaxanthin exists in the form of crystalloid or aliphatic ester is extracted from natural sources or obtained by chemical synthesis. The dosage form of the zeaxanthin added is microencapsulated powder, or homogeneously dispersed in edible vegetable oils, or water dispersible emulsion. Wherein the mass percent of the zeaxanthin in the corresponding dosage form is 1-25 wt%.

Wherein the formula food further contains at least one antioxidant, including synthetic tocopherol (vitamin E), natural tocopherol (natural vitamin E), sodium D-ascorbate (sodium iso-VC), ascorbic acid (vatamine C), ascorbyl palmi- tate, phosphatidylcholine.

Wherein the formula food is applied in various formula milk powder, various functional beverages, various baked food, various dietary supplement tablet and capsule. In particular, the formula food can be used in various food for the purpose of enhancing certain nutritional function, such as formula milk powder, including infant formula milk powder or senile formula milk powder and the like; various functional beverages like beverages containing milk, orange juice beverage, coke, carbonated beverage, suspended beverage, and so on; baked food like cakes, biscuits and so on, and various dietary supplement tablets, capsules and so on for the purpose of nutrition supplement are included as well.

In the present invention, the suitable dosage form of the lutein, zeaxanthin, zinc and selenium to be added should be dissolved in water or oil in advance, and then added during the suitable step of formula food processing.

The addition of the components as described above will not affect the initial taste in the scope of the present invention.

Within the scope of the present invention, the composition of the four substances mixed in a certain proportion can be made into soft capsules or hard capsules or tablets for oral administration thereby achieving the objective of diet supplementing.

In addition, a certain amount of antioxidant such as synthetic tocopherol, natural tocopherol, ascorbyl palmitate; phosphatidylcholine and so on may be added into the composition, or into the single component dosage of lutein, zeaxanthin, zinc, selenium.

The beneficial effect of the present invention is supplementing the mixture of lutein, zeaxanthin, zinc, selenium, and properly increasing the amount of zeaxanthin relative to lutein to improve the effect of the composition on asthenopia remission and improvement of eye consciousness symptoms. The formula food of the present invention is beneficial for human eye health, in particular, for the increase of visuognosis persistence and asthenopia remission and improvement of eye consciousness symptoms.

### BRIEF DESCRIPTION OF THE DRAWINGS

Figure 1 shows the changing rate of visuognosis persistence after feeding trails for the subjects from different test groups.
Figure 2 shows the percentage of the subjects whose visual fatigue is ameliorated in respective test group.

### DETAILED DESCRIPTION AND PREFERRED EMBODIMENTS THEREOF

The features of the present invention will be more clearly understood by reference to the following embodiments, which are not to be construed as limiting the present invention.

### EXAMPLE 1 - Test Groups I -IV not according to the present invention

### Asthenopia Remission Test Of Lutein, Zeaxanthin, Zinc, Selenium

278 subjects, ranging in age between 17-23 years old, who use video display terminals (VDT) for long time and tend to have asthenopia, were selected for this test according to the voluntary principle, with the exception the following subjects: those who have certain ophthalmic disease that may affect the testing results; those who have administered the relevant drugs, health products or applied the other treatments for long or short time that may affect the determination for the results; those who do not confirm to the inclusion criteria, and do not feed the tested drug according to regulations, or have incomplete information and the like that may affect performance or safe-determination.

Control group and test group were established in the test, wherein 48 subjects were randomly selected to administer placebo in control group. The test group was further divided into five groups ( , , , ,), 46 subjects for each group. The subjects in each test group were successively treated with drug compositions in the form of tablets for 35 days, once per day, two tablets once. The test is adopted for the comparison before and after treatment as well as the comparison among groups.

**Table 1 Groups Of Human Test And Compositions Of Feeding Tablets**

| **Table 1 Groups Of Human Test And Compositions Of Feeding Tablets** | |
|---|---|
| Groups | Compositions of feeding tablets |
| Control group | Placebo |
| Test group I | 12mg lutein |
| Test group II | 9mg lutein and 3mg zeaxanthin |
| Test group III | 4mg lutein and 8mg zeaxanthin, |
| Test group IV | 9mg lutein, 3mg zeaxanthin, 27mg zinc and 120µg selenium |
| Test group V | 4mg lutein, 8mg zeaxanthin, 27mg zinc and 120µg selenium |

| | |
|---|---|
| 1. Except the active ingredients shown in the formulation, in which the lutein was Caroltsold' 10% TAB; zeaxanthin was Carolzea' 10% TAB; zinc was zinc ascorbate; selenium was selenoamino acid like L-selenomethionine; the others were necessary for tablets, such as hydroxymethylcellulose, modified starch, and the like. The total weight of the tablet was 450 mg. 2. The appearance, color and weight of placebo tablet were the same as those of feeding tablet. | |

Each index, including physiological index and functional index, was tested once at the beginning and end of the feeding test. Physiological index includes general physical examination, blood, urine, stool routine examination, blood biochemical criterion examination, type-B ultrasound, chest X-ray, and other corresponding examinations like electrocardiographic examination. Functional index includes ocular symptom examination: Inquiring the case history, and observing the subjective symptom of eyes, for example, eye swelling, ophthalmalgia, photophobia, visual blurring, eye dryness and the like. Aggregate the score before and after feeding according to symptoms degree (3 points as severe symptom, 2 points as moderate symptom, 1 points as mild symptom), and calculate the symptom improvement rate according to the improvement of the main symptom (1 point as effective improvement for each symptom, 2 points as obvious improvement). Detection of visuognosis persistence: the percentage of visuognosis duration to fixation duration is referred as visuognosis persistence. The detection duration is 3 min, and the average value of two detections was taken.

Data processing: data were analyzed by using the statistical software STATE6.0. Paired t test was used forself-control data, and group t test was used for comparison between the mean values of two groups. The later one needs homogeneity test for variance to do suitable variable transformation for the data with non-normal distribution or variance nonhomogeneity until the normal variance homogeneity is met, and then the transformed data is used for t test; if the transformed data could not meet the normal variance homogeneity, t test or rank-sum test is used; but for the data whose coefficient of variation is too large, for example, CV>50%, the rank sum test should be used. X2 test was used for performance indexes.

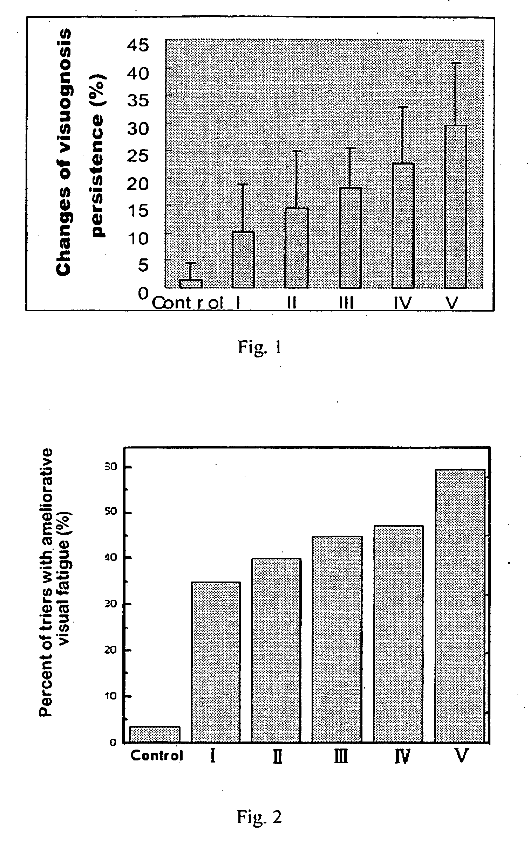

The change of visuognosis persistence for control and test groups after feeding is shown in Figure 1. The visuognosis persistence for subjects in test group was remarkably improved after feeding compared with that before feeding (P<0.05), wherein, it increased 10.27±8.56% for those supplemented with lutein only; the improving extent *10* varied depending on the proportion of lutein to zeaxanthin when the mixture of lutein and zeaxanthin was supplemented, for example, under the condition that the total amount is constant, the visuognosis persistence increased 14.56±10.21% for those supplemented with lutein and zeaxanthin in the proportion of 3:1 and 18.23±7.25% for those supplemented with lutein and zeaxanthin in the proportion of 1:2. There is remarkably difference between the two (P<0.05). When supplemented with a certain amount of trace elements zinc, selenium, the visuognosis persistence of subjects is further promoted, and even the improving extent are more obvious when the proportion of lutein and zeaxanthin was lower (P<0.01).

It can be seen the visual improvement effect from the index of subjective symptom of eyes (clinical symptom score, refer to Figure 2). That is, the results before and after feeding have statistical significance (P<0.001).
Furthermore, when supplemented with the mixture of lutein, zeaxanthin, zinc, selenium, in which the content of zeaxanthin was higher than that of lutein, the highest total efficiency was up to 59.45%, which was remarkably different (P<0.05) from that when the content of lutein was higher (total efficiency was 47.27%).

The results of general physical examination, blood biochemical criterion examination, urine routine examination for control group and test group were within the normal range before and after feeding. The results of chest fluoroscopy,
electrocardiographic, abdominal examination by type-B ultrasound, chest x-ray for control group and test group were within the normal range before and after feeding.

The test proves the advantages of the present invention, that is, supplementing the mixture of lutein, zeaxanthin, zinc, selenium, and properly increasing the amount of zeaxanthin relative to lutein can improve the effect of the composition for asthenopia remission and improvement of eye consciousness symptoms.

### EXAMPLE 2 - not according to the present invention

### The Eye Health Beverage Containing Lutein, Zeaxanthin, Zinc, Selenium

The beverage contained sugar, citric acid, sodium citrate, vitamin B, lutein, zeaxanthin, zinc, selenium, plant extracts, and the like. Based on the total amount of 1000g, 60 mg lutein, 0.3 mg zeaxanthin,40 mg zinc, 30µg selenium are contained thereinto, other components and water are also included. Certain amount of synthetic tocopherol was used as antioxidant. The used lutein was in the form of 5%CWS-B beadlet, and zeaxanthin was in the form of 5%CWS dry powder. The zinc was zinc orotate and selenium was L-selenomethionine.

The beverage was tested through human test according to the test design similar to that of Example 1, and the results are shown in Table 2.

**Table 2 The Comparison Of Change Of Visuognosis Persistence In Two Groups After Feeding With Eye Health Beverage(X±S, %)**

| Groups Before feeding After feeding Promoted percentage | | | |
|---|---|---|---|
| Groups | Before feeding | After feeding | Promoted percentage |
| Test group(n=54) | 53.26±7.87 | 68.48±6.98 | 15.26±9.54^{***###} |
| Control group(n=54) | 52.35±8.01 | 53.47±4.98 | 1.12±4.55^{*} |

Self-comparison before and after feeding, ** P<0.05 ,*** P<0.001 ,Comparison between test group and control group after feeding, ### P<0.001

### EXAMPLE 3 - not according to the present invention

### The Infant Formula Milk Powder Containing Lutein, Zeaxanthin, Zinc, Selenium

Besides conventional carbohydrate, fat, protein, vitamins A, D, E, K, B, trace elements, oligosaccharide, the infant formula milk powder further contained components beneficial for eye health like lutein, zeaxanthin, zinc, selenium. Based on the total amount of 1000g, 0.3 mg lutein, 60 mg zeaxanthin, 4 mg zinc, 150µg selenium are contained thereinto.
The used lutein was in the form of 1%CWS dry powder, and zeaxanthin was in the form of 5%CWS dry powder. The zinc was zinc sulfate; selenium was Baker's yeast rich in selenium; and natural tocopherol was antioxidant.

The formula milk powder was tested through human test according to the test design similar to that of Example 1, and the results are shown in Table 3.

### EXAMPLE 4 - not according to the present invention

### The Milk-Containing Beverage Containing Lutein, Zeaxanthin, Zinc, Selenium

The beverage contained sugar, whole milk powder, sodium citrate, vitamin B, stabilizer, lutein, zeaxanthin, zinc, selenium, and the like. Based on the total amount of 1000g, 250 mg lutein, 12.5 mg zeaxanthin, 80 mg zinc, 180µg selenium, certain amount of ascorbyl palmitate are contained thereinto, other components and water are also be included.
The used lutein was in the form of 10%CWS-B beadlet, and zeaxanthin was in the form of 1%CWS dry powder. The zinc was zinc orotate and selenium was sodium selenophosphate.

The beverage was tested through human test according to the test design similar to that of Example 1, and the results are shown in Table 3.

### EXAMPLE 5

### The Formula Milk Powder For Middle-Aged Persons And Old People Containing Lutein, Zeaxanthin, Zinc, Selenium

Besides conventional carbohydrate, fat, protein, vitamins A, D, E, K, B, trace elements, oligosaccharide, the formula milk powder contained components beneficial for eye health like lutein, zeaxanthin, zinc, selenium. Based on the total amount of 1000g, 12.5 mg lutein, 250 mg zeaxanthin,40 mg zinc, 350µg selenium and a suitable amount of phosphatidylcholine are contained thereinto. The used lutein was in the form of 1%CWS dry powder, and zeaxanthin was in the form of 5%CWS dry powder. The zinc was zinc sulfate; selenium was sodium selenate.

The formula milk powder was tested through human test according to the test design similar to that of Example 1, and the results are shown in Table 3.

### EXAMPLE 6 - not according to the present invention

### The Bread Containing Lutein, Zeaxanthin, Zinc, Selenium

Besides conventional plain and strong flour, sugar, butter for stirring, yeast, certain amount of bread improver, the formula bread contained components beneficial for eye health like lutein, zeaxanthin, zinc, selenium. Based on the total amount of 1000g, 54.7 mg lutein, 19.4 mg zeaxanthin,60 mg zinc, 150µg selenium and a proper amount of sodium D-ascorbate are contained thereinto. The used lutein was in the form of 10%CWS dry powder, and zeaxanthin was in the form of 12%CWS dry powder. The zinc was zinc sulfite; selenium was selenium-enriched Brewer's yeast. The obtained breads have uniform fabric, golden yellow color and flavor taste.

The formula milk powderwas tested through human test according to the test design similar to that of Example 1, and the results are shown in Table 3.

### EXAMPLE 7 - not according to the present invention

### The Fruit Jelly Containing Lutein, Zeaxanthin, Zinc, Selenium

Besides conventional fruit jelly gel, sugar, calcium lactate, citric acid, the formula fruit jelly contained components beneficial for eye health like lutein, zeaxanthin, zinc, selenium, and was also added with certain amount of mixed tocopherol, sodium iso-VC. Based on the total amount of 1000g, 15.0 mg lutein, 1.4 mg zeaxanthin, 40 mg zinc, 250µg selenium are contained thereinto and ascorbic acid was used as antioxidant. The used lutein was in the form of 25%dry powder, and zeaxanthin was in the form of 5%CW8 dry powder. The zinc was zinc orotate; selenium was sodium selenite. The obtained fruit jellies had attracting, lucid, and transparent color.

The formula milk powder was tested through human test according to the test design similar to that of Example 1, and the results are shown in Table 3.

### EXAMPLE 8 - Not according to the present invention

### The Soft Gelatin Capsule Rich In Lutein, Zeaxanthin, Zinc, Selenuim

Based on the weight of 200 mg per soft capsule, the following components are included: 12mg aliphatic ester of lutein, 2mg zeaxanthin, 10mg vitamin E, 15mg vitamin C, 3.6 mg zinc orotate, 24µg sodium selenite, 18mg phosphatidylcholine, 30mg soybean oil, wherein the used aliphatic ester of lutein has the concentrate 20.0%OS and the zeaxanthin was in the form of 25%OS.

The soft gelatin capsule was tested through human test according to the test design similar to that of Example 1, and the results are shown in Table 3.

### EXAMPLE 9 - Not according to the present invention

### The Soft Gelatin Capsule Rich In Lutein, Zeaxanthin, Zinc, Selenuim

Based on the weight of 200 mg per soft capsule, the following components are included: 3.6mg lutein, 12mg zeaxanthin, 8mg vitamin E, 10mg vitamin C, 1.8mg zinc orotate, 32µg sodium selenite, 24mg phosphatidylcholine, 21 mg soybean oil, wherein the used aliphatic ester of lutein has the concentrate 25% OS and the zeaxanthin was in the form of 20%OS.

The soft gelatin capsule was tested through human test according to the test design similar to that of Example 1, and the results are shown in Table 3.

**Table 3 The Changing Rate Of Visuognosis Persistence After Feeding Among Different Groups**

| *Examples* | *Test groups* | | *Control groups* | |
|---|---|---|---|---|
| | *No. of subjects* | *Average changing rate (%)* | *No. of subjects* | *Average changing rate (%)* |
| *Example 3* | *21* | *25.98±8.79** | *19* | *5.87±2.40* |
| *Example 4* | *22* | *8.98±1.56*** | *23* | *-1.51±0.46* |
| *Example 5* | *24* | *14.06±2.85*** | *21* | *3.78±1.58* |
| *Example 6* | *19* | *12.23 ±3.65** | *19* | *3.62±1.76* |
| *Example 7* | *23* | *15.85±8.54** | *24* | *0.84±1.12* |
| *Example 8* | *21* | *13.57±1.69*** | *21* | *0.17±2.03* |
| *Example 9* | *27* | *16.45±5.23*** | *22* | *-1.45±0.74* |

### Comparison with control groups, ^{*} P<0.05, ^{**} P<0.01.

### EXAMPLE 10 - not according to the present invention

### The Bread Containing Lutein, Zeaxanthin, Zinc, Selenium

Besides conventional plain and strong flour, sugar, butter for stirring, yeast, certain amount of bread improver, the formula bread contained components beneficial for eye health like lutein, zeaxanthin, zinc, selenium. Based on the total amount of 1000g, 80.0 mg lutein, 19.4 mg zeaxanthin,60 mg zinc, 150µg selenium and a proper amount of sodium D-ascorbate are contained thereinto. The used lutein was in the form of 10%CWS dry powder, and zeaxanthin was in the form of 12%CWS dry powder. The zinc was zinc sulfite; selenium was selenium-enriched Brewer's yeast. The obtained breads have uniform fabric, golden yellow color and flavor taste.

The formula milk powder was tested through human test according to the test design similar to that of Example 1, and the results are shown in Table 4.

### EXAMPLE 11- Not according to the present invention

### The Soft Gelatin Capsule Rich In Lutein, Zeaxanthin, Zinc, Selenuim

Based on the weight of 250 mg per soft capsule, the following components are included: 40mg aliphatic ester of lutein, 5mg zeaxanthin, 10mg vitamin E, 15mg vitamin C, 3.6 mg zinc orotate, 24µg sodium selenite, 18mg phosphatidylcholine, 20mg soybean oil, wherein the used aliphatic ester of lutein has the concentrate 75% OS and the zeaxanthin was in the form of 20%OS.

The soft gelatin capsule was tested through human test according to the test design similar to that of Example 1, and the results are shown in Table 4.

### EXAMPLE 12 - Not according to the present invention

### The Soft Gelatin Capsule Rich In Lutein, Zeaxanthin, Zinc, Selenuim

Based on the weight of 250 mg per soft capsule, the following components are included: 20mg lutein, 20mg zeaxanthin, 8mg vitamin E, 10mg vitamin C, 1.8 mg zinc orotate, 32µg sodium selenite, 14mg phosphatidylcholine, 26mg soybean oil, wherein the used zeaxanthin was in the form of 25%OS.

The soft gelatin capsule was tested through human test according to the test design similar to that of Example 1, and the results are shown in Table 4.

### EXAMPLE 13 - Not according to the present invention

### Tablet Rich In Lutein, Zeaxanthin, Zinc, Selenuim

Based on the weight of 250mg per Tablet, the following components are included: 40mg aliphatic ester of lutein, 5mg zeaxanthin, 10mg vitamin E, 15mg vitamin C, 3.6mg zinc orotate, 24µg sodium selenite, 18mg phosphatidylcholine, 20mg soybean oil, wherein the used aliphatic ester of lutein has the concentrate 75% OS and the zeaxanthin was in the form of 20%OS.

The soft gelatin capsule was tested through human test according to the test design similar to that of Example 1, and the results are shown in Table 4.

**Table 4 The Changing Rate Of Visuognosis Persistence After Feeding Among Different Groups**

| Examples | Test groups | | Control groups | |
|---|---|---|---|---|
| | No. of subjects | Average changing rate (%) | No. of subjects | Average changing rate (%) |
| Example 10 | 19 | 12.53±3.55* | 19 | 3.52±1.76 |
| Example 11 | 21 | 13.87±1.59** | 21 | 0.27±2.03 |
| Example 12 | 27 | 16.85±5.33** | 22 | -1.55±0.64 |
| Example 13 | 21 | 13.77±1.69** | 21 | 0.27±2.03 |

| | | | | |
|---|---|---|---|---|
| Comparison with control groups, * P<0.05, ** P<0.01. | | | | |

## Claims

1. A formula food beneficial for eye health, comprises lutien, zeaxanthin, zinc, and selenium; wherein the amount of the lutein added is 0.3∼250mg/kg in the formula food; the amount of the zeaxanthin added is 0.3∼250mg/kg in the formula food; the amount of the zinc added is 4∼80mg/kg in the formula food; the amount of the selenium added is 30∼350µg/kg in the formula food; the proportion of mass of lutein and zeaxanthin is in the range of 0.1:2.0∼1.0:1.0..

2. The formula food according to the claim 1, wherein the amount of the lutein added is 2.0∼80mg/kg in the formula food; the amount of the zeaxanthin added is 2.0∼80mg/kg in the formula food.

3. The formula food according to the claim 1 or 2, wherein the lutein is extracted from natural sources and exists in the form of crystalloid or aliphatic ester.

4. The formula food according to the claim 3, wherein the dosage form of the lutein added is microencapsulated powder, or homogeneously dispersed in edible vegetable oils, or aqueous dispersible emulsion.

5. The formula food according to any one of claims 1∼4, wherein the mass percent of the lutein in the corresponding dosage form is 1∼25 wt%.

6. The formula food according to any one of claims 1∼2, wherein the zeaxanthin is extracted from natural sources or obtained by chemical synthesis and exists in the form of crystalloid or aliphatic ester.

7. The formula food according to claim 6, wherein the dosage form of the zeaxanthin added is microencapsulated powder, or homogeneously dispersed in edible vegetable oils, or aqueous dispersible emulsion.

8. The formula food according to any one of the claims 1∼2, wherein the mass percent of zeaxanthin in the corresponding dosage form is 1∼25 wt%.

9. The formula according to the claim 1, wherein the zinc is inorganic zinc salt or organic zinc salt, wherein the inorganic zinc salt is zinc sulfate or zinc sulfite, the organic zinc salt is zinc orotate or zinc ascorbate.

10. The formula food according to the claim 1, wherein the selenium is inorganic selenium salt or organic selenium salt; wherein the inorganic selenium salt is selenophosphate, or selenite; the organic selenium salt is selenoamino acid or selenium yeast or selenium-enriched yeast.

11. The formula food according to the claim 10, wherein the selenophosphate is sodium selenophosphate; the selenite is sodium selenite or sodium selenate; the selenoamino acid is L-selenomethionine; the selenium yeast or selenium-enriched yeast is Brewer's yeast or Barker's yeast.

12. The formula food according to the claim 1, wherein the formula food further comprises at least one antioxidant, the antioxidant comprises synthesized tocopherol, natural tocopherol, sodium D-ascorbate, ascorbic acid, ascorbyl palmitate, phosphatidylcholine.

13. A use of the formula food according to any one of claims 1∼12 in the preparation of various formula milk powders, various functional beverages, various baked food, various dietary supplement tablets and capsules.

## Patentansprüche

1. Eine für die Visuognose förderliche Lebensmittelzusammensetzung, welche Lutein, Zeaxanthin, Zink und Selen umfasst, wobei die Menge an zugesetztem Lutein in der Lebensmittelzusammensetzung zwischen 0,3 und 250 mg/kg liegt, die Menge an zugesetztem Zeaxanthin in der Lebensmittelzusammensetzung zwischen 0,3 und 250 mg/kg liegt, die Menge an zugesetztem Zink in der Lebensmittelzusammensetzung zwischen 4 und 80 mg/kg liegt, die Menge an zugesetztem Selen in der Lebensmittelzusammensetzung zwischen 30 und 350 µg/kg liegt, wobei das Massenverhältnis von Lutein zu Zeaxanthin im Bereich von 0,1:2,0 bis 1,0:1,0 ist.

2. Lebensmittelzusammensetzung nach Anspruch 1, bei welcher die Menge an zugesetztem Lutein in der Lebensmittelzusammensetzung zwischen 2,0 und 80 mg/kg und die Menge an zugesetztem Zeaxanthin in der Lebensmittelzusammensetzung zwischen 2,0 und 80 mg/kg liegt.

3. Lebensmittelzusammensetzung nach Anspruch 1 oder 2, bei welcher das Lutein aus natürlichen Quellen extrahiert wird und in Form eines kristalloiden oder aliphatischen Esters vorliegt.

4. Lebensmittelzusammensetzung nach Anspruch 3, bei welcher die Dosierung des zugesetzten Luteins in Form von mikroverkapseltem Pulver oder homogen dispergiert in tierischen Speiseölen oder als wässrige dispergierbare Emulsion erfolgt.

5. Lebensmittelzusammensetzung nach irgend einem der Ansprüche 1 bis 4, bei welcher der prozentuale Massenanteil des Luteins in der entsprechenden Dosierungsform zwischen 1 und 25 Gew.-% liegt.

6. Lebensmittelzusammensetzung nach irgend einem der Ansprüche 1 bis 2, bei welcher das Zeaxanthin aus natürlichen Quellen extrahiert wird oder durch chemische Synthese erhalten wird und in Form eines kristalloiden oder aliphatischen Esters vorliegt.

7. Lebensmittelzusammensetzung nach Anspruch 6, bei welcher die Dosierung des zugesetzten Zeaxanthin in Form von mikroverkapseltem Pulver oder homogen dispergiert in tierischen Speiseölen oder als wässrige dispergierbare Emulsion erfolgt.

8. Lebensmittelzusammensetzung nach irgend einem der Ansprüche 1 bis 2, bei welcher der prozentuale Massenanteil des Zeaxanthin in der entsprechenden Dosierungsform zwischen 1 und 25 Gew.-% liegt.

9. Lebensmittelzusammensetzung nach Anspruch 1, bei welcher das Zink anorganisches Zinksalz oder organisches Zinksalz ist und das anorganische Zinksalz Zinksulfat oder Zinksulfit ist und das organische Zinksalz Zinkorotat oder Zinkascorbat ist.

10. Lebensmittelzusammensetzung nach Anspruch 1, bei welcher das Selen anorganisches Selensalz oder organisches Selensalz ist, wobei das anorganische Selensalz Selenphosphat oder Selenit ist und das organische Selen Selenaminosäure oder Selenhefe oder mit Selen angereicherte Hefe ist.

11. Lebensmittelzusammensetzung nach Anspruch 10, bei welcher das Selenphosphat Natriumselenphosphat ist, das Selenit Natriumselenit oder Natriumselenat sind und die Selenaminosäure L-Selenmethonin ist und die Selenhefe oder die mit Selen angereicherte Hefe Bierhefe oder Barker-Hefe ist.

12. Lebensmittelzusammensetzung nach Anspruch 1, bei welcher die Lebensmittelzusammensetzung außerdem mindestens einen Antioxidanten umfasst, wobei dieser Antioxidant synthetisch hergestelltes Tocopherol, natürliches Tocopherol, Natrium-D-Ascorbat, Ascorbinsäure, Ascorbylpalmitat und Phosphatidylcholin umfasst.

13. Anwendung der Lebensmittelzusammensetzung nach irgend einem der Ansprüche 1 bis 12 bei der Zubereitung von verschiedenen Milchpulverzusammensetzungen, verschiedenen funktionellen Getränken, verschiedenen Backwaren, verschiedenen Nahrungsergänzungsmitteln in Form von Tabletten und Kapseln.

## Revendications

1. Formulation alimentaire bénéfique pour la santé oculaire, comprenant de la lutéine, de la zéaxanthine, du zinc et du sélénium, dans laquelle la quantité de lutéine ajoutée est de 0,3 à 250 mg/kg dans la formule alimentaire ; la quantité de zéaxanthine ajoutée est de 0,3 à 250 mg/kg dans la formule alimentaire ; la quantité de zinc ajouté est de 4 à 80 mg/kg dans la formule alimentaire ; la quantité de sélénium ajouté est de 30 à 350 µg/kg dans la formule alimentaire ; la proportion en poids de la lutéine et de la zéaxanthine est de l'ordre de 0,1:2,0 à 1.0:1.0.

2. Formulation alimentaire selon la revendication 1, dans laquelle la quantité de lutéine ajoutée est de 2,0 à 80 mg/kg dans la formulation alimentaire, la quantité de zéaxanthine ajoutée est de 2,0 à 80 mg/kg dans la formulation alimentaire.

3. Formulation alimentaire selon la revendication 1 ou 2, dans laquelle la lutéine est extraite de sources naturelles et existe sous la forme d'ester cristalloïde ou aliphatique.

4. Formulation alimentaire selon la revendication 3, dans laquelle la forme galénique de la lutéine ajoutée est une poudre micro-encapsulée ou dispersée de façon homogène dans des huiles végétales comestibles ou une émulsion aqueuse dispersible.

5. Formulation alimentaire selon l'une quelconque des revendications 1 à 4, dans laquelle le pourcentage en poids de la lutéine dans la forme galénique correspondante est de 1 à 25 % en poids.

6. Formulation alimentaire selon l'une quelconque des revendications 1 à 2, dans laquelle la zéaxanthine est extraite de sources naturelles ou obtenue par synthèse chimique et existe sous forme d'ester cristalloïde ou aliphatique.

7. Formulation alimentaire selon la revendication 6, dans laquelle la forme galénique de la zéaxanthine ajoutée est une poudre micro-encapsulée ou dispersée de façon homogène dans des huiles végétales comestibles ou une émulsion aqueuse dispersible.

8. Formulation alimentaire selon l'une quelconque des revendications 1 à 2, dans laquelle le pourcentage en poids de zéaxanthine dans la forme galénique correspondante est de 1 à 25 % en poids.

9. Formulation selon la revendication 1, dans laquelle le zinc est un sel de zinc inorganique ou un sel de zinc organique, dans laquelle le sel de zinc inorganique est le sulfate de zinc ou le sulfite de zinc, le sel de zinc organique est l'orotate de zinc ou l'ascorbate de zinc.

10. Formulation alimentaire selon la revendication 1, dans laquelle le sélénium est un sel de sélénium inorganique ou un sel de sélénium organique ; dans laquelle le sel de sélénium inorganique est le sélénophosphate ou le sélénite ; le sel de sélénium organique est l'acide séléno-aminé ou la levure de sélénium ou une levure enrichie en sélénium.

11. Formulation alimentaire selon la revendication 10, dans laquelle le sélénophosphate est le sélénophosphate de sodium ; le sélénite est le sélénite de sodium ou le sélénate de sodium ; l'acide séléno-aminé est la L-sélénométhionine ; la levure de sélénium ou la levure enrichie en sélénium est la levure de bière ou la levure de boulanger.

12. Formulation alimentaire selon la revendication 1, la formulation alimentaire comprenant en outre au moins un antioxydant, l'antioxydant comprenant un tocophérol synthétisé, un tocophérol naturel, du D-ascorbate de sodium, de l'acide ascorbique, du palmitate d'ascorbyle, de la phosphatidylcholine.

13. Utilisation de la formulation alimentaire selon l'une quelconque des revendications 1 à 12, dans la préparation de diverses formules de lait en poudre, diverses boissons fonctionnelles, divers aliments cuits au four, divers comprimés et capsules de compléments alimentaires.
